# DEMANDE DE BREVET EUROPEEN

(11) **EP 1 048 306 A2**
(43) Date de publication de la demande: **02.11.2000**
(21) Numéro de dépôt: 00500081.5
(22) Date de dépôt: 28.04.2000
(51) Int. Cl.: A61M 1/02

(54) **Ensemble de dérivation pour poches à sang**

(30) Priorité: 30.04.1999 ES 9901126 U
(71) Demandeur: Arrontes Caballero, Enrique, 28027 Madrid (ES)
(72) Inventeur: Arrontes Caballero, Enrique, 28027 Madrid (ES)

(57) **Abrégé**

Ensemble de dérivation pour l'extraction et réinfusion en bourses de sang, caractérisé pour avoir une unique dérivation en Y avec le tuyau principal (7), ou bien une conection multiple en Y avec le tuyau principal (11,12,13).

L'angle de conection du tuyau (7) ou des tuyaux (11,12,13) avec la voie principale aura un angle très aigu (9).

Le point de termination distale de la voie auxilière (7), sera de type standard femelle (Luer-Lock femelle) enroulé (8,10).

Les points de termination distales des voies auxilières (11,12,13), seront de type standard femelle enroulé (Luer-Lock femelle) (8,10), ou quelqu'un deux d'un type de matériale perforable (14).

## Description

### ANTÉCÉDENTS:

Jusqu'au moment les équipes de bourses de sang adoptent, basiquement deux esquèmes: a) ce qui respondent au model classique, auxquel l'union de l'aiguille qui s' inserte au bras du donneur avec la termination dans la bourse de sang de recueil, est droit sans dérivations et, b) ce qui dans le traject antérieurment décrit, ont une dérivation en Y, finie, des fois en une aiguille protégée et des autres en un point perforable.

Si bien la modalité de la derivation en Y a signifié un avantage respect au sistème classique, parce qu'il n'a pas besoin de couper le tuyau d'union pour l'extraction des échantillons pour analyse, ce qui meilleure l'image devant le donneur, l'hygiène et la qualité de l'échantillon, par contre il oblige à l'utilization de sistèmes d'extraction sous vide et, encore, il resulte impossible la réinfusion à travers de cette voie de n'importe qui fluide que le donneur peuve préciser, pour présenter un acontéciment advers.

L'antérieur suppose une meilleure dans l'extraction des échantillons pour être analysées et vérifier l'idonité du donneur mais, comme inconvénient, figurent: l'imposibilité de réinfuser les fluids par cette voie auxilière et d'être obligatoire l'utilization des sistèmes sous vides pour l'extraction des échantillons.

### EXPLICATION DE L'INVENTION:

Ce présent Model d'Utilité solucionne les problèmes antérieurment décrit parce qu'il comprends une dérivation en Y respect au tuyau principal (d'union aguille- bourse), qui doit avoir un angle avec le même, qui ne provoque pas de turbulences dans le sang qui fluit par la voie principale jusqu'arriver à la bourse de sang.

Ce tuyau auxilière de dérivation en Y, pourra être unique ou de dispositif multiple, il doit avoir un sistème similaire à une pince externe incorporée qui empêche son remplir pendant le temps qui n'intérèse pas.

Le mencioné tuyau auxilière finira en une conexion standard femelle (Luer-Lock, femelle), ce qui permet l'extraction des échantillons: qu'il se fasse avec seringue, qu'ils s'éxtrairent les quantitées exactes qui se nécessitent, en conditions d'hygiène et maxime propreté, sans nécessité de couper la voie principale avec le ciseau, en permettant le remplir de cette voie auxilière quand réalement il se nécessite (avec la pince externe), sans contaminer avec l'anticoagulant (parce que l'extraction par cette voie se fait au final de l'extraction quand la bourse de sang c'est déjà remplit, en ferment le pas à la voie principale), sans risque de contamination pour les échantillons témoins ni pour le sang contenu dans la bourse.

Tout l'antérieur représente le compliment des normes plus estrictes de l'Organization Mondiale de la Santé, la minimization des coûts en face des autres sistèmes, simplicité du méthode en face d'autres, la meilleure de la qualité des échantillons, l'évitation des risques d'accidents au personnel sanitaire qui manipule l'équipe d'extraction, assurance pour le donneur et les échantillons, accomplissement des récomendations du Conseil d'Europe.

Finalement la termination de la voie de dérivation auxilère en Y (qui peut être unique ou multiple), en finissant en conexion standard femelle (Luer-Lock,femelle), en plus de toutes les utilitées exposées au paragraf antérieur, elle vient fermée avec le bouchon enroulé qui garantise éviter le risque de contamination, seulement il se désenroule quand il va se connecter à la seringue, la voie se dépince se qui permet la remplir de la même et il s'extrait la quantité nécessaire, en passant de nouveau au pinceau et en enroulant la même quand le procédiment est fini.

En plus de l'antérieur, en cas de préciser la réinfusion de quelque fluide, en inclusant la prope unité de sang nouvellement donnée, ça suffit avec la conexion d'un sistème conventionel d'infusion, tous sont du type standard mâle (Luer-Lock, mâle), ce qui en cas de réaction adverse au donneur, nous avons la voie chanalisée parce que nous ne l'avons pas perdu et nous pouvons l'utiliser tout le temps qui soit précis pour l'infusion des réqueriments à travers de cette voie auxilière.

Le présent dessin permet, à travers la conexion décrite (voie auxilière en Y finie en conexion standard femelle -Luer-Lock, femelle-), la posibilité d'extraction de plusieurs bourses de sang en utilisant une unique picûre au bras du donneur, espécialement si le dispositif a la possibilité, antérieurment décrite au présent Model d'Utilité, d'être des conexions multiples. Quelqu'une des mêmes peut être simplement un point d'extraction des dejá connus de matériel perforable à travers d'aiguille.

Pour une meillure compréhension, ont adjoint les schèmes qui représentent les dispositifs décrits au présent Model d'Utilité.

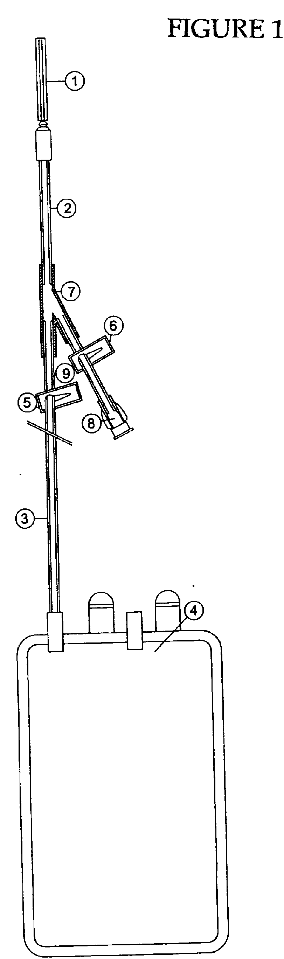

Figure n° 1: Elle représente une vision frontale du dispositif dans son ensemble, avec une unique dérivation en Y.

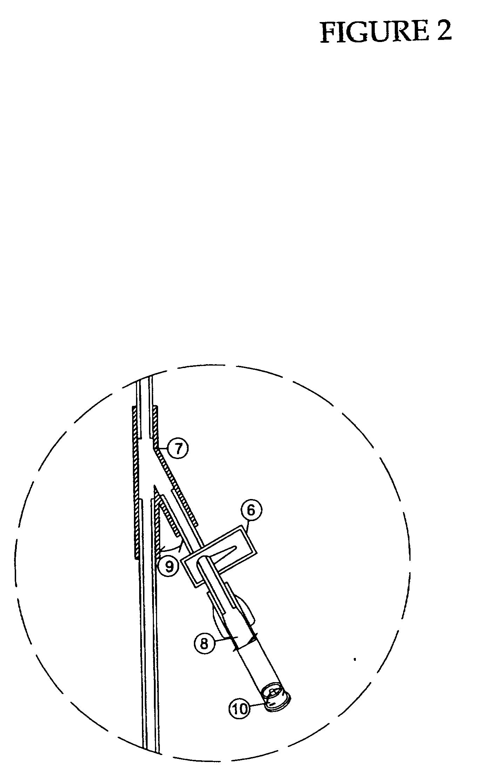

Figure n° 2: Elle représente une vision frontale prochaine du dispositif en Y qui contient la termination object du présent Model d'Utilité.

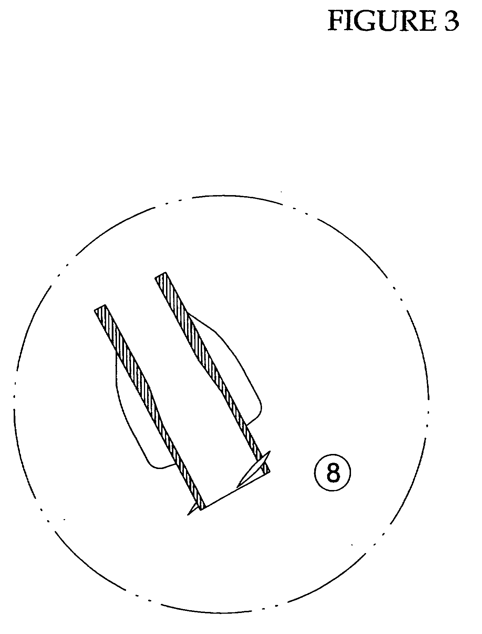

Figure n°3: Vision de la termination standard femelle (Luer-Lock femelle).

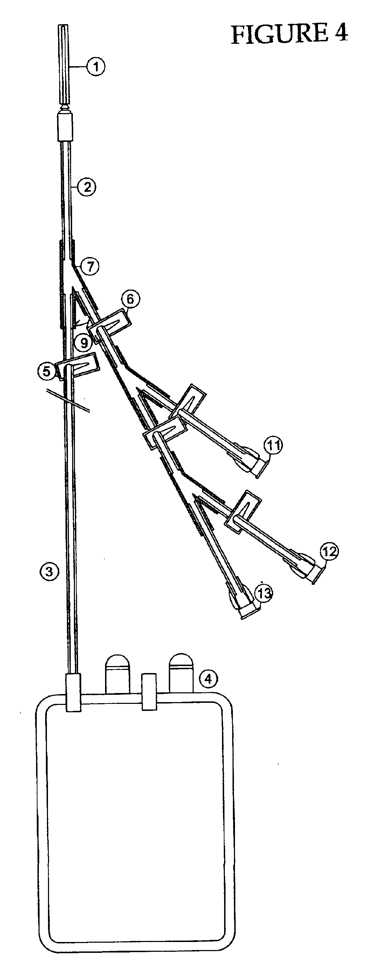

Figure n°4: Elle représente une vision frontale d'un sistème qui a de multiples conexions, lesquelles finissent en conexion standard femelle (Luer Lock femelle).

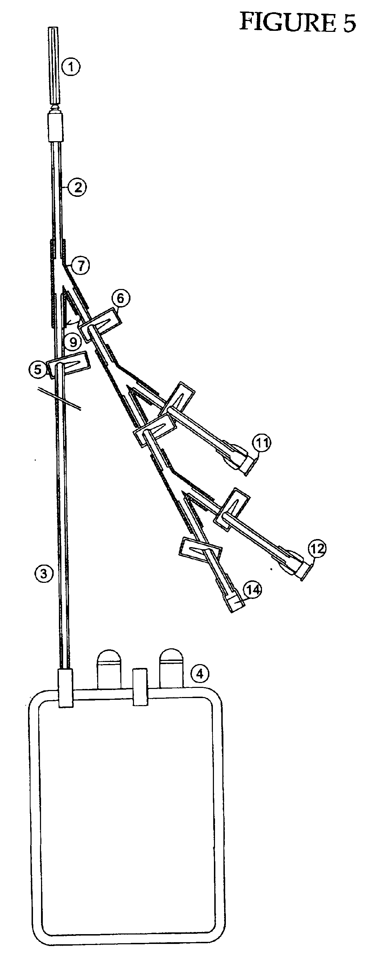

Figure n°5 : Elle représente un dessin multiple quelque des terminales sont seulement des points de picûre de matériel perforable avec aiguille.

Tel et comme il se représente dans les figures, le sistème object du présent Model d'Utilité, reste constituit fondamentalment par:
(1) L'aiguille qui s'inserte au bras du donneur
(2) Portion proximale du tuyau de conexion aiguille-bourse ou tuyau principal
(3) Portion distale du tuyau de conexion aiguille-bourse ou tuyau principal
(4) Bourse de sang, qui recueille la donation efectuée par le donneur
(5) Pince située au tuyau principal, toujours distale à la conexion en Y
(6) Pince située au tuyau auxilière de derivation en Y
(7) Tuyau auxilière de dérivation en Y
(8) Termination standard femelle (Luer Lock femelle) du tuyau auxilière de dérivation en Y, avec bouchon remuable
(9) Angle entre le tuyau principal et le tuyau auxilière de dérivation en Y
(10) Bouchon remuable de la termination standard femelle (Luer Lock femelle) du tuyau auxilière de dérivation en Y
(11) (12) (13) Terminations multiples standard femelle (Luer Lock femelle) des tuyaux auxilières de dérivation en Y, en cas d'être un dispositif de multiples dérivations
(14) Termination en point perforable, pour quelqu'une des terminations de quelque tuyau auxilière de dérivation en Y, aux dispositifs de multiples dérivations. Tout ce qui n'affecte pas, n'altère pas, ne change pas ou ne modifique pas, l'ésence de ce dispositif décrit, sera variable aux effects de ce Model d'Utilité.

## Revendications

1. Ensemble de dérivation pour l'extraction et réinfusion en bourses de sang, caracterisé pour posseder un tuyau de section circulaire, en matérial plastique ou similaire, conecté en Y avec la voie principale d'une bourse de sang, telle conexion en Y pourra être d'un unique terminale ou induire plusieurs dérivations en Y.

2. Ensemble de dérivation pour l'extraction et réinfusion en bourses de sang, selon revendication première, caracterisé par la conexion avec la voie principale, ell doit avoir un angle très aigu, de façon qui ne produce pas de turbulences dans le sang. La voie principale, dans la zone de conexion avec la dérivation en Y se pourra réaliser avec une pièce auxiliaire.

3. Ensemble de dérivation pour léxtraction et réinfusion en bourses de sang, selon revendication première, caracterisé par, quand le même soit de dérivation unique, il utilise une termination standard femelle (Luer-Lock femelle), avec un bouchon enroulé.

4. Ensemble de dérivation pour l'extraction et réinfusion en bourses de sang, selon revendication première, caracterisé par, quand le même soit de dérivation multiple, tous les terminales ou quelqu'un sera de type standard femelle (Luer-Lock femelle), avec un bouchon enroulé et, le reste des terminales seront de matérial perforable avec aiguille.
